# FASCICULE DE BREVET EUROPEEN

(11) **EP 3 145 948 B1**
(45) Date de publication et mention de la délivrance du brevet: **01.01.2020**
(21) Numéro de dépôt: 15732775.0
(22) Date de dépôt: 20.05.2015
(51) Int. Cl.: C07K 14/415, C12N 15/82

(54) **PRODUCTION COMMERCIALE DE L'ALLERGÈNE AMB A1 PAR EXPRESSION TRANSITOIRE CHEZ LES PLANTES**
KOMMERZIELLE HERSTELLUNG VON ALLERGEN AMB A 1 MITTELS TRANSIENTER EXPRESSION IN PFLANZEN
COMMERCIAL PRODUCTION OF ALLERGEN AMB A 1 BY MEANS OF TRANSIENT EXPRESSION IN PLANTS

(30) Priorité: 23.05.2014 FR 1454653
(43) Date de publication de la demande: 29.03.2017
(73) Titulaire: Angany Inc., Québec, QC G1R 1R2 (CA)
(72) Inventeur: GOMORD, Véronique, 76000 Rouen (FR); FITCHETTE, Anne-Catherine, 76570 Pavilly (FR); CATALA, Virginie, 27950 Saint Marcel (FR); FAYE, Loïc, 76160 St Jacques sur Darnetal (FR)
(74) Mandataire: Plasseraud IP
(86) Numéro de dépôt international: PCT/FR2015/051319
(87) Numéro de publication internationale: WO 2015/177464

(56) Documents cités:
- EP-A1- 2 692 732
- WO-A2-00/20612
- FR-A1- 2 809 413
- GRIFFITH I J ET AL: "SEQUENCE POLYMORPHISM OF AMB A I AND AMB A II, THE MAJOR ALLERGENS IN AMBROSIA ARTEMISIIFOLIA (SHORT RAGWEED)", INTERNATIONAL ARCHIVES OF ALLERGY AND APPLIED IMMUNOLOGY, BASEL, CH, vol. 96, no. 4, 1 janvier 1991 (1991-01-01), pages 296-304, XP000647685, ISSN: 0020-5915
- GREGORY P. POGUE ET AL: "Production of pharmaceutical-grade recombinant aprotinin and a monoclonal antibody product using plant-based transient expression systems", PLANT BIOTECHNOLOGY JOURNAL, vol. 8, no. 5, 1 juin 2010 (2010-06-01), pages 638-654, XP055038517, ISSN: 1467-7644, DOI: 10.1111/j.1467-7652.2009.00495.x
- VOINNET O ET AL: "An enhanced transient expression system in plants based on suppression of gene silencing by the p19 protein of tomato bushy stunt virus", THE PLANT JOURNAL, BLACKWELL SCIENTIFIC PUBLICATIONS, OXFORD, GB, vol. 33, no. 5, 1 mars 2003 (2003-03-01), pages 949-956, XP002367694, ISSN: 0960-7412, DOI: 10.1046/J.1365-313X.2003.01676.X
- LOÏC FAYE ET AL: "Success stories in molecular farming-a brief overview", PLANT BIOTECHNOLOGY JOURNAL, vol. 8, no. 5, 9 mai 2010 (2010-05-09), pages 525-528, XP055038398, ISSN: 1467-7644, DOI: 10.1111/j.1467-7652.2010.00521.x
- Jos Cantillo ET AL: "From Molecular Cloning to Vaccine Development for Allergic Diseases" In: "An Integrated View of the Molecular Recognition and Toxinology - From Analytical Procedures to Biomedical Applications", 1 juillet 2013 (2013-07-01), InTech, XP055161355, ISBN: 978-9-53-511151-1 DOI: 10.5772/52821,

## Description

La présente invention se rapporte à une cellule végétale comprenant une molécule d'ADN comprenant au moins une séquence nucléotidique hétérologue codant pour une préproprotéine d'une pectate lyase choisie parmi Amb a 1, la sous-unité alpha d'Amb a 1, la sous-unité bêta d'Amb a 1, et les homologues d'Amb a 1, liée de manière fonctionnelle à un promoteur fort, ladite molécule d'ADN n'étant pas intégrée dans le génome de la cellule végétale. L'invention se rapporte également un procédé de production de la pectate lyase utilisant la cellule végétale.

L'ambroisie, en particulier *Ambrosia artemisiifolia,* est une plante annuelle, qui appartient à la famille des Composées tubuliflores (ou Astéracées). Cette plante sauvage, originaire d'Amérique du nord, fleurit de la fin de l'été jusqu'à l'automne. Son pollen a un pouvoir allergénique très élevé. Ainsi, l'ambroisie est la première cause d'allergie pollinique aux Etats-Unis, avec environ 30 millions de personnes sur 40 millions souffrant de pollinoses. Introduite involontairement en France au XIXème siècle, l'ambroisie est une plante fortement invasive qui a rapidement progressé à partir de la région Rhône-Alpes vers le nord de la France. Ainsi, en 2011, des pieds d'ambroisie ont été retrouvés dans l'Ile de France.

Le pollen de cette plante provoque chez les personnes sensibles une réaction allergique caractérisée par sa sévérité. Globalement, la rhinite allergique à l'ambroisie est beaucoup plus sévère que celle causée par d'autres pollens. Les symptômes tels que prurit, anosmie (perte ou diminution de la sensibilité aux odeurs), rhinorrhée, éternuements et obstruction nasale sont plus importants. De plus, dans 50% des cas, cette allergie évolue en un asthme, souvent plus sévère que celui provoqué par d'autres pollens. Ces symptômes sont d'autant plus prononcés que le taux de pollen dans l'air est élevé.

Dans la région Rhône-Alpes, l'allergie à l'ambroisie touche aujourd'hui 6 à 12% de la population entre fin août et début septembre. Ainsi, pour cette seule région, le nombre total de consommateurs de médicaments antiallergiques à l'ambroisie a fortement augmenté (+60%) entre 2008 et 2011, passant de 161 200 à 258 700 personnes, et les dépenses de santé engendrées par cette allergie représentent entre 5,6 et 8,6 millions d'euros. L'allergie à l'ambroisie représente donc aujourd'hui un véritable problème de santé publique. Le plus simple pour se protéger des risques d'allergie serait d'éviter les expositions au pollen d'ambroisie, mais une éviction totale semble d'ores et déjà impossible malgré des arrêtés préfectoraux rendant obligatoire la destruction de l'ambroisie. Ces mesures n'ont pas permis d'enrayer la propagation de cette plante invasive dans un grand nombre de régions.

Les traitements dits « symptomatiques » soit par voie locale (gouttes, collyres, sprays), soit par voie générale (comprimés, gélules), permettent de soulager les symptômes pendant la période où les pollens sont présents dans l'air. Mais les symptômes reviennent rapidement dès l'arrêt du traitement. A l'inverse des traitements "symptomatiques", l'immunothérapie allergénique (ou désensibilisation) est le seul traitement susceptible de modifier l'évolution de la maladie allergique. Cette voie thérapeutique permet de traiter la cause de l'allergie contrairement aux médicaments dits « symptomatiques ». Toutefois, comme pour les autres allergies, les extraits utilisés aujourd'hui pour la désensibilisation des patients allergiques à l'ambroisie sont d'une qualité insuffisante pour que ce traitement soit efficace. Plusieurs allergènes d'ambroisie ont été caractérisés, en particulier Amb a 1 qui est responsable de plus de 80% des cas d'allergie à l'ambroisie. Différentes tentatives de production d'un Amb a 1 recombinant destiné à une désensibilisation spécifique ont échoué. En effet, Amb a 1 est une protéine végétale complexe présentant des maturations post-traductionelles typiquement végétales, et seul un système d'expression végétale sera capable de produire cet allergène sous une forme utilisable et efficace en immunothérapie allergénique.

Il existe donc un besoin pour produire cet allergène Amb a 1 sous forme recombinante, avec une bonne qualité, et de façon reproductible et efficace (avec un bon rendement). Par « bonne qualité », on entend que l'allergène est très fortement similaire, dans sa séquence et sa structure, à l'allergène présent naturellement dans l'ambroisie. Il existe également un besoin pour produire cet allergène à des niveaux commercialement et économiquement acceptables.

De façon surprenante, les inventeurs ont maintenant découvert que la production de l'allergène Amb a 1, bien que toxique *in vivo,* sous forme recombinante est possible, par expression transitoire chez les plantes. Cette production, qui se fait dans une cellule végétale, permet l'obtention de l'allergène Amb a 1 sous forme mature et active, avec un bon rendement, de façon très simple et reproductible. En outre, l'allergène Amb a 1 obtenu n'est pas contaminé par des protéases et/ou d'autres contaminants d'origine végétale. Les inventeurs ont ainsi pu obtenir une production, à des niveaux commercialement acceptables, de l'allergène Amb a 1.

L'invention a donc pour objet une cellule végétale comprenant une molécule d'ADN comprenant au moins une séquence nucléotidique hétérologue codant pour une préproprotéine d'une pectate lyase choisie parmi Amb a 1, la sous-unité alpha d'Amb a 1, la sous-unité bêta d'Amb a 1, et les homologues d'Amb a 1, liée de manière fonctionnelle à un promoteur fort, de préférence un promoteur 35S. La molécule d'ADN liée au promoteur fort permet d'exprimer de façon transitoire la pectate lyase mature et active dans la cellule. De préférence, ladite cellule végétale est une cellule de *Nicotiana benthamiana.* De préférence, la cellule végétale selon l'invention comprend une molécule d'ADN comprenant au moins une séquence nucléotidique hétérologue codant pour une préproprotéine de la pectate lyase liée de manière fonctionnelle à un promoteur fort, de préférence un promoteur 35S, ladite molécule d'ADN n'étant pas intégrée dans le génome de la cellule végétale.

La pectate lyase choisie parmi Amb a 1, la sous-unité alpha d'Amb a 1, la sous-unité bêta d'Amb a 1, et les homologues d'Amb a 1 est appelée « pectate lyase selon l'invention » dans la présente demande.

L'invention a également pour objet une plante comprenant au moins une cellule végétale selon l'invention.
Un autre objet de l'invention est de fournir un procédé de production d'une pectate lyase, comprenant l'expression de ladite pectate lyase dans une cellule végétale selon l'invention, ou dans une plante comprenant une telle cellule.
Une pectate lyase susceptible d'être obtenue par le procédé selon l'invention, avec une cellule végétale selon l'invention, ou avec une plante selon l'invention peut être utilisée comme médicament. Elle peut également être utilisée en immunothérapie allergique, seule ou en combinaison avec au moins un autre allergène d'Asteracée. Elle peut également être utilisée dans le diagnostic d'allergies, et être intégrée dans un kit de diagnostic d'allergies.

La cellule végétale selon l'invention comprend une molécule d'ADN liée de manière fonctionnelle à un promoteur fort, de préférence un promoteur 35S. Par « lié de manière fonctionnelle », on entend que la molécule d'ADN est fusionnée avec le promoteur fort, de telle sorte que le promoteur fort induise la transcription de la molécule d'ADN.

Cela permet l'expression de ladite molécule d'ADN sous forme transitoire, i.e. sans intégration de l'ADN, notamment l'ADNc, dans le génome de la cellule végétale. En effet, l'utilisation de l'expression transitoire dans une cellule végétale ou une plante selon l'invention permet d'augmenter les rendements de production de la pectate lyase selon l'invention sous forme active jusqu'à des niveaux élevés, compatibles avec une exploitation commerciale, mais incompatibles avec la survie d'une plante qui exprimerait ces pectates lyases toxiques de façon stable. Dans le cas d'une expression transitoire, la récolte de la biomasse végétale a lieu lors du pic d'expression de la protéine recombinante, i.e. typiquement 4 à 6 jours après la transfection.
De préférence, la cellule végétale selon l'invention comprend un vecteur d'expression comprenant au moins une séquence nucléotidique hétérologue codant pour une préproprotéine d'une pectate lyase selon l'invention liée de manière fonctionnelle à un promoteur fort, de préférence un promoteur 35S.

Amb a 1 est une pectate lyase provenant d'*Ambrosia artemisiifolia.* Reconnu par plus de 80 % des patients sensibilisés à l'ambroisie, cet allergène est responsable de plus de 90 % de l'activité allergénique du pollen d'ambroisie. C'est une protéine de 38 kDa décrite comme non glycosylée appartenant à la famille des pectates lyases. Douze isoformes de Amb a 1 sont recensées, de Amb a 1.0101 à Amb a 1.0502 (d'après le site www.allergome.org).
Les séquences nucléique et d'acides aminés des douze isoformes sont détaillées dans le tableau 1 ci-dessous :

**Tableau 1 : les douze isoformes d'Amb a 1, leurs séquences nucléiques et protéiques**

| **Isoallergènes** | **N° d'accession (GenBank) Séquence nucléique** | **N° d'accession (UniProt) Séquence d'acides aminés** |
|---|---|---|
| Amb a 1.0101 | M80558 | P27759 |
| Amb a 1.0201 | M62981 | P27760 |
| Amb a 1.0202 | FR669658 | E1XUL3 |
| Amb a 1.0301 | M62961 | P27761 |
| Amb a 1.0302 | | P27761 (variant L48Y) |
| Amb a 1.0303 | M80560 | P27761 (variant H392R) |
| Amb a 1.0304 | FR669659 | E1XUL4 |
| Amb a 1.0305 | FR669660 | E1XUL5 |
| Amb a 1.0401 | M80562 | P28744 |
| Amb a 1.0402 | FR669664 | E1XUL9 |
| Amb a 1.0501 | M80561 | P27762 |
| Amb a 1.0502 | FR669666 | E1XUM1 |

Par « Amb a 1 » selon l'invention, on entend les douze isoformes précitées dans le tableau 1 ci-dessus. Les séquences protéiques décrites dans ce tableau 1 correspondent aux préproprotéines des différentes isoformes.

Amb a 1 subit une protéolyse dans le grain de pollen et/ou au cours du processus d'extraction ou de purification résultant en deux chaînes, alpha (26 kDa) et bêta (12 kDa), associées de façon non covalente (King et al, 1974, 1981). Il a été démontré que des modifications chimiques d'Amb a 1, y compris la réduction et l'alkylation des ponts disulfures ainsi que le processus de dénaturation/renaturation par l'urée ou la succinylation des résidus lysine, réduit sa réactivité vis-à-vis des IgE (King, 1976; Smith et al, 1988). Les sous-chaînes alpha et bêta d'Amb a 1 ont une réactivité différente sur les IgE et les lymphocytes T. En effet, Amb a 1 bêta contient un nombre important d'épitopes fixant les IgE, tandis que Amb a 1 alpha se comporte comme un hypoallergène et stimule l'activité des cellules T.

De préférence, Amb a 1 a pour séquence protéique SEQ ID NO :7 (préproprotéine). De préférence, la sous-unité bêta a pour séquence protéique SEQ ID NO :8. De préférence, la sous-unité alpha a pour séquence protéique SEQ ID NO :9.

Par « homologues d'Amb a 1 », on entend des protéines de la famille des Astéracées qui présentent au moins 57% d'identité, de préférence au moins 60% d'identité, de préférence au moins 64% d'identité, avec l'une des isoformes d'Amb a 1. De préférence, l'homologue d'Amb a 1 est une protéine de plante du genre *Ambrosia* ou *Artemisia,* plus préférentiellement d'*Artemisia vulgaris,* d'*Ambrosia psilostachya* ou d'*Ambrosia trifida,* qui présente au moins 57% d'identité, de préférence au moins 60% d'identité, de préférence au moins 64% d'identité, avec l'une des isoformes d'Amb a 1. De préférence, l'homologue d'Amb a 1 est choisi parmi Amb p 1 (provenant d'*Ambrosia psilostachya,* de code d'accession 9064 sur www.allergome.org) et Art v 6 (provenant d*'Artemisia vulgaris,* de numéro d'accession A0PJ16 dans Uniprot).

Par "pourcentage d'identité" entre deux séquences d'acides aminés au sens de la présente invention, on entend désigner un pourcentage de résidus d'acides aminés identiques entre les deux séquences à comparer, obtenu après le meilleur alignement, ce pourcentage étant purement statistique et les différences entre les deux séquences étant réparties au hasard et sur toute leur longueur. Par "meilleur alignement" ou "alignement optimal", on entend l'alignement pour lequel le pourcentage d'identité déterminé comme ci-après est le plus élevé.

Les comparaisons de séquences entre deux séquences d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981, J. Mol Evol., 18:38-46), au moyen de l'algorithme d'homologie locale de Neddleman et Wunsch (1970), au moyen de la méthode de recherche de similarité de Pearson et Lipman (1988, PNAS, 85: 2444-2448), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI).

Ainsi, de préférence, la pectate lyase selon l'invention est choisie parmi Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, la sous-unité alpha d'Amb a 1, la sous-unité bêta d'Amb a 1 et les protéines de la famille des Astéracées qui présentent au moins 57% d'identité, de préférence au moins 60% d'identité, de préférence au moins 64% d'identité, avec l'une des isoformes d'Amb a 1.
Plus préférentiellement, la pectate lyase selon l'invention est choisie parmi Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, la sous-unité alpha d'Amb a 1, la sous-unité bêta d'Amb a 1 et les protéines d'*Artemisia vulgaris,* d'*Ambrosia psilostachya* ou d'*Ambrosia trifida,* qui présentent au moins 57% d'identité, de préférence au moins 60% d'identité, de préférence au moins 64% d'identité, avec l'une des isoformes d'Amb a 1.

La molécule d'ADN comprend au moins une séquence nucléotidique hétérologue codant pour une préproprotéine de la pectate lyase selon l'invention.
La préproprotéine de la pectate lyase selon l'invention comprend un peptide signal, un propeptide et la pectate lyase mature. La séquence nucléotidique hétérologue codant la préproprotéine selon l'invention comprend donc une séquence codant le peptide signal, une séquence codant le propeptide et une séquence codant la pectate lyase mature. De préférence, la séquence nucléotidique hétérologue codant la préproprotéine selon l'invention comprend une séquence codant le peptide signal, une séquence codant le propeptide de la sous-unité bêta, une séquence codant la sous-unité bêta, une séquence codant le propeptide de la sous-unité alpha et une séquence codant la sous-unité alpha.
Le peptide signal est notamment tout peptide signal reconnu par la cellule végétale, provenant ou non d'une pectate lyase. Il cible notamment la pectate lyase selon l'invention dans le milieu intercellulaire. De préférence, le peptide signal est celui de la chitinase de tabac, ou le peptide signal naturel de la pectate lyase.
Le propeptide est, quant à lui, le propeptide naturel d'une pectate lyase, ou bien le propeptide d'une peptidase C1A. Dans un mode de réalisation préféré, le propeptide est un propeptide de peptidases C1A, notamment d'acariens ou d'origine végétale. De préférence, le propeptide de peptidase C1A est le propeptide naturel ou le propeptide muté ou non de Der p1. De préférence, le propeptide de peptidase C1A est choisi parmi le propeptide naturel de peptidase C1A et la séquence SEQ ID NO :6 (i.e. les acides aminés 19 à 98 de la séquence Uniprot p08176). Dans un mode de réalisation préférée de la présente invention, la pectate lyase selon l'invention est produite en fusion avec son propre propeptide ou en fusion avec le propeptide de peptidase C1A d'acariens ou en fusion avec un propeptide de peptidase C1A végétale, afin d'augmenter les rendements de production de la protéine.

De préférence, la séquence de la préproprotéine de pectate lyase est choisie parmi les séquences protéiques listées dans le tableau 1 ci-avant et les séquences présentant au moins 57%, de préférence au moins 60%, de préférence au moins 64% d'identité avec l'une de ces dernières.

De préférence, la séquence nucléotidique hétérologue codant la préproprotéine est choisie parmi SEQ ID NO :1 à 5. De préférence, elle a pour séquence SEQ ID NO :1, SEQ ID NO :2, SEQ ID NO :3, SEQ ID NO :4 ou SEQ ID NO :5.

Selon l'invention, la séquence nucléotidique hétérologue codant la préproprotéine peut être obtenue à partir d'un gène connu déjà cloné codant pour une pectate lyase selon l'invention, ou bien par criblage d'une banque d'ADNc avec des anticorps anti-pectate lyase. Les méthodes utilisées sont bien connues de l'homme de l'art, et incluent notamment l'identification du gène par hybridation avec des sondes, PCR, séquençage et clonage moléculaire. Il est également possible de synthétiser le gène pour refléter l'utilisation des codons préférés chez les plantes ; dans ce cas, on parle d'optimisation de codons, souvent utile pour une forte expression des protéases sélectionnées (Murray et al, Nucleic Acid Res. 17:477 498 (1980)).

Dans un mode de réalisation préféré de l'invention, la séquence nucléotidique hétérologue codant la préproprotéine comprend en outre une séquence, appelée séquence d'adressage intracellulaire. Cette séquence permet de cibler un compartiment de stockage de la pectate lyase selon l'invention, afin de contrôler sa maturation. Cette maturation est nécessaire pour l'obtention des pectates lyases sous la forme la plus adaptée pour le diagnostic d'allergie et l'immunothérapie. De préférence, cette séquence peptidique d'adressage cible la pectate lyase selon l'invention sous forme soluble ou membranaire vers le réticulum endoplasmique ou les différents compartiments constitutifs du système endomembranaire de sécrétion de la cellule végétale.

Une fois que le gène d'intérêt a été isolé et modifié pour contenir tout ou partie des modifications décrites ci-dessus et obtenir la séquence nucléotidique hétérologue, cette dernière est placée dans un vecteur d'expression par des méthodes classiques. La sélection d'un vecteur d'expression approprié dépendra de la méthode d'introduction du vecteur d'expression dans des cellules hôtes. Un vecteur d'expression typique contient des éléments d'ADN procaryote codant pour une origine de réplication bactérienne et un gène de résistance à un antibiotique à fournir pour la croissance et la sélection du vecteur d'expression dans l'hôte bactérien, un site de clonage pour l'insertion d'une séquence d'ADN exogène codant pour la pectate lyase; des éléments d'ADN eucaryotes comme une séquence d'initiation de la transcription du gène exogène, comme un promoteur, et des éléments d'ADN qui contrôlent le traitement des transcrits, comme des séquences de terminaison / polyadénylation et une cassette d'expression permettant l'expression d'un inhibiteur du silencing. Il contient également des séquences telles que des t-DNA qui sont nécessaires pour l'intégration d'un morceau d'ADN dans la plante ou dans la cellule végétale.
De préférence, le vecteur d'expression comprend :
- des éléments d'ADN procaryote codant pour une origine de réplication bactérienne et un gène de résistance à un antibiotique ;
- au moins une séquence nucléotidique hétérologue codant pour une préproprotéine d'une pectate lyase selon l'invention liée de manière fonctionnelle à un promoteur fort, de préférence un promoteur 35S ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos (séquence de terminaison de la nopaline synthase).
De préférence, le vecteur d'expression est pAG01.

Les promoteurs utilisés pour contrôler l'expression de la pectate lyse sont des promoteurs forts, et peuvent être des promoteurs de gènes de plantes, tels que par exemple, le promoteur de l'ubiquitine, le promoteur de la petite sous-unité de la ribulose-1, 5-bis-phosphate carboxylase, des promoteurs *d'Agrobacterium tumefaciens,* les promoteurs de la nopaline-synthase et de l'octopine synthase, ou encore des promoteurs viraux comme les 19S et 35S du virus de la mosaïque du chou-fleur (CaMV). De préférence, le promoteur fort est le 35S.

L'expression élevée et la qualité des pectates lyases recombinantes produites dans l'invention permettent de concevoir leur production commerciale. En effet, la pectate lyase selon l'invention est typiquement exprimée en une quantité égale à au moins 0,1 % des protéines solubles totales de la plante, mais souvent en une quantité beaucoup plus élevée, pouvant atteindre 5 à 10%.

La plante comprenant les cellules végétales selon l'invention peut être une plante entière, mais peut également être une partie de plante, telle que des feuilles.

La plante ou les cellules végétales selon l'invention peuvent être utilisées en tant que telles, comme médicament.
La plante ou les cellules végétales selon l'invention peuvent être utilisées en tant que telles, pour des applications comme biocarburant, dans la nutrition animale, ou encore dans la production de papier ou de textile (fibres de coton notamment).
Dans d'autres applications, la pectate lyase recombinante est purifiée après extraction à partir de la plante ou des cellules végétales qui l'expriment. Afin de faciliter sa purification, l'enzyme pourra être exprimée en fusion avec des tags (His6, GST, MBP, FLAG etc..) qui seront localisés de préférence en position N- ou C-terminale de la protéine mature.

La pectate lyase susceptible d'être obtenue par le procédé selon l'invention, avec une cellule végétale selon l'invention, ou avec une plante selon l'invention, peut être utilisée comme médicament. Elle peut également être utilisée en immunothérapie allergique, seule ou en combinaison avec au moins un autre allergène d'Asteracée. Cet autre allergène d'Asteracée peut être choisi parmi Amb a 11, Amb a 5 et leurs mélanges.
Elle peut également être utilisée dans le diagnostic d'allergies, notamment d'allergies aux pollens. La présente description divulgue donc également un kit de diagnostic d'allergies, comprenant la pectate lyase susceptible d'être obtenue par le procédé selon l'invention, avec une cellule végétale selon l'invention, ou avec une plante selon l'invention, et des moyens de mesure des anticorps dirigés contre cette protéine. Est également décrit un kit de diagnostic d'allergies, comprenant la pectate lyase susceptible d'être obtenue par le procédé selon l'invention, avec une cellule végétale selon l'invention, ou avec une plante selon l'invention, des moyens de mesure des anticorps dirigés contre cette protéine, et des moyens de mesure des anticorps dirigés contre un autre allergène d'Asteracée, par exemple l'allergène Amb a 11 ou Amb a 5.

Les méthodes générales de culture de plantes, ainsi que les procédés pour introduire des vecteurs d'expression dans un tissu végétal, sont à la disposition de l'homme de l'art. Ils sont variés et dépendent de la plante sélectionnée. De préférence, les plantes seront cultivées selon les techniques propres à la plateforme Allergopur. Ce procédé de production de protéines recombinantes est décrit dans la demande FR1255510, et comprend une première étape de culture de la plante, en aéroponie ou en hydroponie, de préférence culture sur flotteur libre, et sous éclairage LEDs. Après cette première étape, notamment cinq semaines de culture en hydroponie sur des flotteurs libres, l'agroinfiltration des plantes est réalisée sous vide, par des agrobactéries comprenant un fragment d'ADN codant pour la pectate lyase selon l'invention. Cette étape d'agroinfiltration peut être mise en œuvre par tout moyen permettant de faire le vide. De préférence, dans le procédé utilisé selon l'invention, elle est réalisée sous vide par effet Venturi. Parmi les agrobactéries utilisables selon l'invention, on peut citer de préférence, les souches LBA4404, GV3101, EHA 101/105 ou C58. Selon une première alternative, une fois l'étape d'agroinfiltration réalisée, les plantes sont typiquement égouttées tête en bas pendant 15 minutes, puis remises en culture, typiquement 3 à 6 jours, idéalement en assurant une brumisation fréquente de ces dernières pendant les 6 premières heures de culture qui suivent l'agroinfiltation. Enfin, la protéine est extraite et purifiée. De préférence, la protéine est extraite et purifiée comme décrit dans la demande FR1255510. Selon une seconde alternative, une fois l'étape d'agroinfiltration réalisée, les plantes sont directement remises en culture, typiquement 3 à 6 jours, puis la protéine est extraite et purifiée.
L'extraction de la protéine peut être effectuée par broyage des feuilles, ou encore selon un procédé faisant appel à une infiltration enzymatique.
Dans un tel procédé faisant appel à une infiltration enzymatique, l'extraction de la protéine est effectuée par les étapes suivantes :
- infiltration sous vide (notamment comme il a été décrit ci-dessus pour l'agroinfiltration) de la partie aérienne des plantes, dans une solution enzymatique contenant une pectinase ou de la macérozyme, qui ne présente pas d'activité protéolytique. De préférence, la pectinase est la P162L commercialisée par Biocatalyst, formulée à 4% dans un milieu comprenant 50mM de citrate de sodium pH5.2, 0.5M NaCl et 0.04% métabisulfite. De préférence, la macérozyme est formulée à 0.5% dans un milieu comprenant 50mM de citrate de sodium pH5.2, 0.5M NaCl et 0.04% métabisulfite,prélèvement des feuilles des plantes infiltrées, et incubation dans une solution enzymatique de pectinase ou de macérozyme, pendant une durée comprise entre 2h30 et 5h, de préférence de 3h ou 4h30, à une température comprise entre 24°C et 30°C, de préférence 26°C, puis
- mise sous agitation du mélange obtenu entre 20 et 30 rpm à température ambiante (i.e. environ 20-23°C) pendant une durée comprise entre 30 minutes et 2h,
- le digestat est ensuite filtré, de préférence sur une toile de 250µm, puis éventuellement centrifugé (par exemple à 250xg pendant 10 minutes),
- le surnageant est récupéré afin d'effectuer une filtration en profondeur de préférence sur filtre K100 (commercialisé par Pall). De préférence, le filtrat est concentré 20 fois sur une cassette 5kDa et son pH est ajusté à 7 avec du Na3PO4.
Les allergènes Amb a 1 et Amb a 11 peuvent être purifiés par chromatographie à partir des extraits comme il a été décrit dans la demande FR 1255510.

Ainsi, de préférence, l'invention a également pour objet un procédé de production d'une pectate lyase dans une cellule végétale ou une plante, comprenant les étapes suivantes :
a) transformation d'agrobactéries avec un vecteur d'expression comprenant une séquence nucléotidique hétérologue codant pour une préproprotéine d'une pectate lyase selon l'invention liée de manière fonctionnelle à un promoteur fort ; et
b) transfection de la cellule végétale ou de la plante avec les agrobactéries obtenues à l'étape a).

De préférence, les agrobactéries utilisables dans l'étape a) sont choisies parmi les souches LBA4404, GV3101, EHA 101/105 et C58. De préférence, le vecteur d'expression utilisé dans l'étape a) comprend :
- des éléments d'ADN procaryote codant pour une origine de réplication bactérienne et un gène de résistance à un antibiotique ;
- au moins une séquence nucléotidique hétérologue codant pour une préproprotéine d'une pectate lyase selon l'invention liée de manière fonctionnelle à un promoteur fort, de préférence un promoteur 35S ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos.
La transformation de l'étape a) se fait typiquement par des méthodes connues de l'art antérieur, par exemple par chocs thermiques avec passages successifs à 4°C, -80°C et 37°C.

La transfection de l'étape b) comprend de préférence les étapes suivantes :
b1) culture de la cellule végétale ou de la plante, en aéroponie ou en hydroponie, et sous éclairage LEDs, de préférence pendant cinq semaines en hydroponie sur des flotteurs libres,
b2) agroinfiltration de la cellule végétale ou plante obtenue en b1) sous vide, par les agrobactéries obtenues à l'étape a). Cette étape d'agroinfiltration est, de préférence, réalisée sous vide par effet Venturi.
b3) remise en culture de la cellule végétale ou de la plante obtenue en b2), typiquement pendant 3 à 6 jours.

Enfin, de préférence, la pectate lyase ainsi produite est extraite et purifiée.
De préférence, le procédé de production d'une pectate lyase selon l'invention dans une cellule végétale ou une plante, comprend en outre une étape c) d'extraction de la pectate lyase produite, ladite étape c) comprenant les étapes suivantes :
- infiltration sous vide de la cellule végétale ou des feuilles (i.e. partie aérienne) de la plante, dans une solution enzymatique contenant une pectinase ou de la macérozyme, qui ne présente pas d'activité protéolytique. De préférence, la pectinase ou la macérozyme sont telles que décrites ci-avant,
- prélèvement de la cellule végétale infiltrée ou des feuilles de la plante infiltrées et incubation, dans une solution enzymatique de pectinase ou de macérozyme, pendant une durée comprise entre 2h30 et 5h, à une température comprise entre 24°C et 30°C, puis

- mise sous agitation du mélange obtenu entre 20 et 30 rpm à température ambiante pendant une durée comprise entre 30 minutes et 2h,
- filtration puis éventuellement centrifugation du digestat obtenu, et
- récupération du surnageant.

Les exemples qui suivent, illustrent, mais ne visent pas à limiter la portée de l'invention. Il sera évident pour l'homme de l'art que des variantes et modifications sont possibles.

Les légendes des figures sont les suivantes :

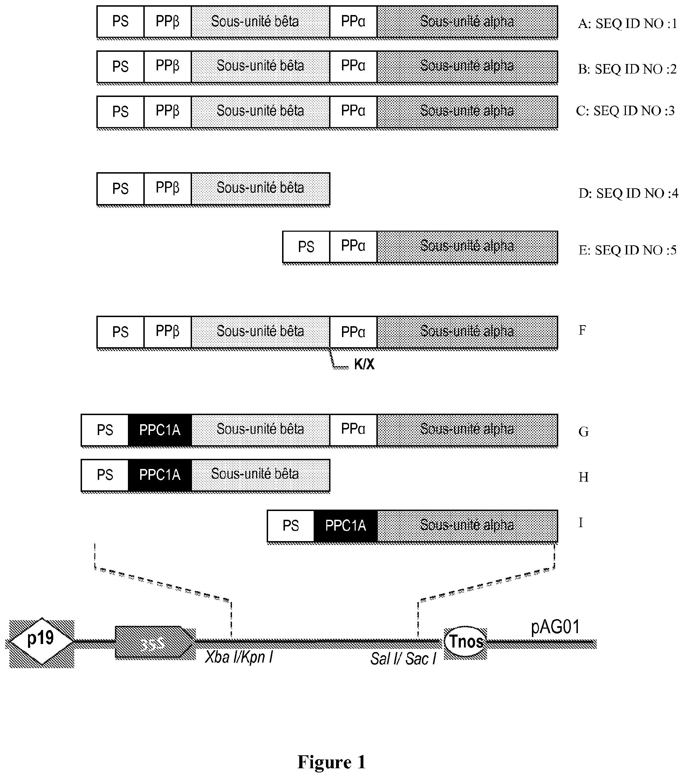

**Figure 1** **: Représentation schématique des différentes cassettes permettant de produire une pectate lyase Amb a 1** mature et active (A, B, C, F et G) ou la sous-unité alpha (E et I) ou la sous-unité bêta (D et H), en utilisant soit le peptide signal et le propeptide naturel de la protéine (A-F), soit le peptide signal de la chitinase de tabac et un propeptide de la famille des peptidases C1A (G-I) afin d'augmenter les rendements.
   La pectate lyase est également produite sous forme mutée sur la lysine 180 (K180) afin de limiter la protéolyse permettant la production des sous-unités alpha et bêta (F).
   A (SEQ ID NO :1): ADNc codant pour la préproprotéine naturelle (de séquence SEQ ID NO :7). Cet ADNc pourra être fusionné à des signaux d'adressage décrits dans la demande WO2008/056265,
   B (SEQ ID NO : 2): ADNc optimisé pour une utilisation chez *N.benthamiana,* codant pour la préproprotéine naturelle (de séquence SEQ ID NO :7). Cet ADNc pourra être fusionné à des signaux d'adressage décrits dans la demande WO2008/056265,
   C (SEQ ID NO : 3): ADNc harmonisé codant pour la forme la préproprotéine naturelle (de séquence SEQ ID NO :7). Cet ADNc contient des codons optimisés pour une utilisation chez *N.benthamiana,* mais comprend également des codons rares, afin de préserver le rythme de synthèse de la protéine pour une meilleure conservation de la structure 3D. Cet ADNc pourra être fusionné à des signaux d'adressage décrits dans la demande WO2008/056265,
   D (SEQ ID NO : 4) : ADNc natif/optimisé/harmonisé codant pour la sous-unité bêta (de séquence SEQ ID NO :8). Cet ADNc pourra être fusionné à des signaux d'adressage décrits dans la demande WO2008/056265.
   E (SEQ ID NO : 5) : ADNc natif/optimisé/harmonisé codant pour la sous-unité alpha (de séquence SEQ ID NO :9). Cet ADNc pourra être fusionné à des signaux d'adressage décrits dans la demande WO2008/056265.
   F : ADNc natif/optimisé/harmonisé codant pour la forme mutée (K180) de la protéine. Cet ADNc pourra être fusionné à des signaux d'adressage décrits dans la demande WO2008/056265.
   G : ADNc natif/optimisé/harmonisé codant pour la forme mature de la protéine fusionnée à la séquence signal de la chitinase de tabac (Neuhaus, J.-M., 1996) et au propeptide Der p1 (p08176 - aa 19 à 98 ou SEQ ID NO :6). Cet ADNc pourra être fusionné à des signaux d'adressage décrits dans la demande WO2008/056265.
   H : ADNc natif/optimisé/harmonisé codant pour la sous-unité bêta de la protéine fusionnée à la séquence signal de la chitinase de tabac (Neuhaus, J.-M., 1996) et au propeptide Der p1 (p08176 - aa 19 à 98 ou SEQ ID NO :6). Cet ADNc pourra être fusionné à des signaux d'adressage décrits dans la demande WO2008/056265.
   I : ADNc natif/optimisé/harmonisé codant pour la sous-unité alpha de la protéine fusionnée à la séquence signal de la chitinase de tabac (Neuhaus, J.-M., 1996) et au propeptide Der p1 (p08176 - aa 19 à 98 ou SEQ ID NO :6). Cet ADNc pourra être fusionné à des signaux d'adressage décrits dans la demande WO2008/056265.

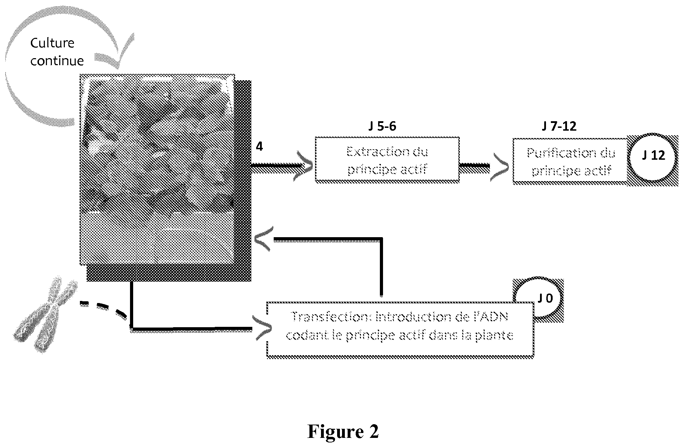

**Figure 2** **: La plateforme AllergoPur** utilisée pour l'expression et la production des différentes formes de pectate lyase selon l'invention.

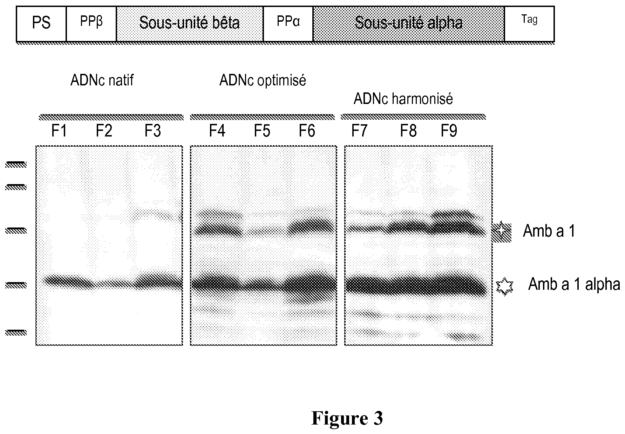

**Figure 3****: Expression de la protéine Amb** a 1. Les protéines extraites de plantes transfectées par l'ADNc natif (F1-F3), par l'ADNc optimisé (F4-F6) ou par l'ADNc harmonisé (F7-F9) codant la protéine Amb a 1 ont été séparées par SDS-PAGE et analysées immmuno-détection avec un anticorps dirigé contre une étiquette Flag. L'analyse par immuno-détection met en évidence la production spécifique de la protéine Amb a 1 et de la sous-unité alpha. La sous-unité bêta clivée n'est pas immunodétectée sur ce blot.

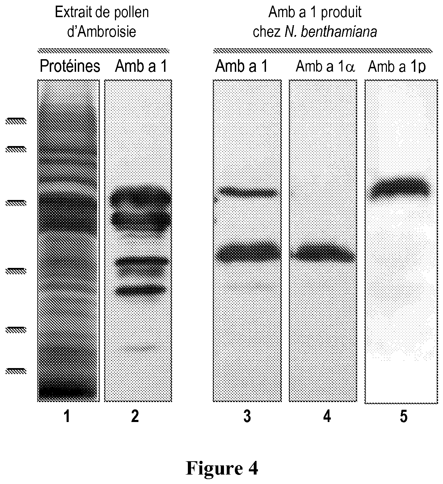

**Figure 4****: Purification de l'allergène Amb a 1.** Les protéines extraites de plantes ont été transfectées sous vide avec l'ADNc harmonisé codant la protéine Amb a 1 par chromatographie IMAC. Puis les fractions retenues sur la colonne ont été analysées par électrophorèse SDS-PAGE. Compte tenu de la position C-terminale du Flag, deux polypeptides correspondant à la forme précurseur (préproprotéine) (Amb a 1p) et à la sous-unité alpha sont purifiés à partir de l'extrait transfecté par l'ADNc complet (piste 3). Ces deux polypeptides (Amb a 1 alpha et Amb a 1p) sont ensuite séparés (pistes 4 et 5 respectivement) par chromatographie de tamisage moléculaire. Les deux polypeptides produits présentent une mobilité électrophorétique légèrement plus faible que les protéines natives. Cette différence s'explique par la présence du flag en position C-terminale.

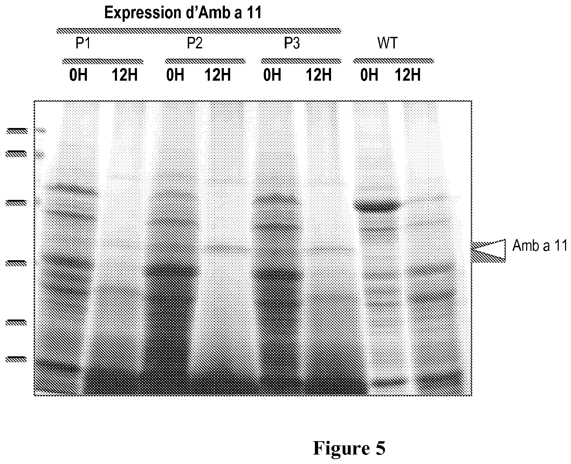

**Figure 5** **: Expression de l'allergène Amb a 11.** Les protéines extraites de 3 plantes (P1 - P3) transfectées par l'ADNc codant l'allergène Amb a 11 ont été séparées par électrophorèse SDS-PAGE. Les extraits protéiques sont analysés dès leur extraction (0H) ou après une incubation de 12h (12H) à température ambiante. L'incubation à température ambiante met en évidence l'accumulation de deux polypeptides correspondant à Amb a 11, ainsi que la dégradation quasi-totale des protéines de *N.benthamiana.* L'allergène Amb a 11 est donc produit sous forme active selon le même procédé que celui décrit présentement pour Amb a 1.

### EXEMPLE :

### Design moléculaire et synthèse de gène

Les ADNc sont synthétisés sous forme native, en optimisant l'usage des codons pour leur reconnaissance par le système végétal ou en harmonisant l'usage de codons (réintroduction de codons rares pour rythmer la synthèse de la protéine). Dans le cadre de cette invention, l'optimisation préférée est l'optimisation pour une expression chez *Nicotiana benthamiana,* comme indiqué en Figure 1.

### Préparation des plasmides

Des sites de restriction Xba I/kpn I et Sal I/Sac I sont respectivement intégrés aux extrémités 5' et 3' de l'ADNc lors de la synthèse. Ces sites sont ensuite utilisés afin de cloner les ADNc dans le vecteur binaire d'expression pAG01 (Figure 1). Les ADNc sont clonés en amont d'un promoteur 35S (35S) et en aval d'une séquence de terminaison de la nopaline synthase (Tnos) ; le vecteur pAG01 contient en outre une cassette d'expression permettant d'exprimer l'inhibiteur de silencing p19 simultanément de la protéine recombinante afin d'augmenter les rendements de production. Les vecteurs sont ensuite utilisés pour transformer la souche LBA4404 *d'Agrobacterium tumefaciens.*

### Expression transitoire de pectates lyases selon l'invention dans des feuilles de Nicotiana benthamiana - Utilisation de la plateforme AllergoPur

Pour la production par expression transitoire *Agobacterium tumefaciens LBA4404* est utilisé pour le transfert d'un ADNc codant la pectate lyase sans que le gène d'intérêt soit intégré dans le génome de la cellule végétale : on parle ici de transfection et non de transgenèse. Les plantes sont cultivées en condition hydroponique en présence d'un milieu nutritif (GHE, floragrow, floramicro, florabloom, 10mL/ 15mL/ 5mL pour 10 L d'eau osmosée) et sous éclairage LED.

L'agrobactérie est transférée dans le tissu foliaire par agro-infiltration selon deux procédés. Pour la production de petits lots de pectates lyases destinés au criblage de prototypes, les agrobactéries sont injectées manuellement grâce à une seringue appliquée contre l'épiderme de la face inférieure de la feuille. Des disques foliaires prélevés dans les feuilles 4 à 6 jours après l'agro-infiltration sont utilisés pour l'analyse des différents prototypes de pectates lyases. Cette étape de criblage permet de définir le vecteur d'expression qui sera utilisé pour l'obtention d'une pectate lyase de qualité optimale. Le même procédé est utilisé pour la production commerciale à grande échelle mais, dans ce cas, l'agro-infiltration s'effectue sous vide, dans des enceintes contenant plusieurs litres d'une culture d'agrobactéries et où plusieurs dizaines de plantes sont infiltrées simultanément. Ces plantes sont ensuite remises en culture pendant 4 à 6 jours avant la purification des pectates lyases à partir des extraits foliaires (Figure 2).

### Expression de la pectate lyase Amb a 1 et de ses sous-unités

L'expression des protéines ainsi que les rendements sont respectivement analysés par Western blotting et ELISA. Les résultats sont illustrés pour les 3 formes d'ADNc codant la protéine naturelle (Figure 3).

Les protéines exprimées sont actives. En effet, l'expression d'Amb a 1 provoque des nécroses importantes dès le 4^{ème} jour d'expression comparé à des plantes contrôles. Ces nécroses sont dues à une forte activité pectate lyase (Liu et al, 2010).

### Purification et caractérisation

Comme l'illustre la figure 4, les pectates lyases sont extraites à partir de la biomasse fraîche ou congelée puis purifiées sur colonne IMAC (HisTrap Excell).

Cette purification permet la production de la forme précurseur (préproprotéine) et de la sous-unité alpha.

Enfin, comme le montre la figure 5, il est possible de combiner les allergènes Amb a 1 et Amb a 11 obtenus par le procédé selon l'invention, pour obtenir une composition utilisable en immunothérapie allergique. Ces allergènes peuvent être produits et purifiés après une extraction mécanique comme décrit dans la demande FR1255510, ou selon l'alternative d'extraction par infiltration enzymatique comme décrit dans la présente demande.

## Revendications

1. Cellule végétale comprenant une molécule d'ADN comprenant au moins une séquence nucléotidique hétérologue codant pour une préproprotéine d'une pectate lyase choisie parmi Amb a 1, la sous-unité alpha d'Amb a 1, la sous-unité bêta d'Amb a 1, et les homologues d'Amb a 1, liée de manière fonctionnelle à un promoteur fort, dans laquelle ladite molécule d'ADN n'est pas intégrée dans le génome de la cellule végétale.

2. Cellule végétale selon la revendication 1, dans laquelle la pectate lyase est choisie parmi Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, la sous-unité alpha d'Amb a 1, la sous-unité bêta d'Amb a 1 et les protéines de la famille des Astéracées qui présentent au moins 57% d'identité, de préférence au moins 60% d'identité, de préférence au moins 64% d'identité, avec l'une des isoformes d'Amb a 1.

3. Cellule végétale selon l'une des revendications 1 ou 2, dans laquelle la pectate lyase est choisie parmi Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, la sous-unité alpha d'Amb a 1, la sous-unité bêta d'Amb a 1 et les protéines d'*Artemisia vulgaris,* d'*Ambrosia psilostachya* ou d'*Ambrosia trifida,* qui présentent au moins 57% d'identité, de préférence au moins 60% d'identité, de préférence au moins 64% d'identité, avec l'une des isoformes d'Amb a 1.

4. Cellule végétale selon l'une des revendications 1 à 3, dans laquelle la séquence nucléotidique hétérologue comprend une séquence codant le peptide signal, une séquence codant le propeptide et une séquence codant la pectate lyase mature.

5. Cellule végétale selon la revendication 4, dans laquelle :
- le peptide signal est choisi parmi le peptide signal naturel de la pectate lyase et le peptide signal de la chitinase de tabac ; et
- le propeptide est choisi parmi les propeptides naturels de pectates lyases et les propeptides de peptidases C1A.

6. Cellule végétale selon l'une des revendications 1 à 5, dans laquelle la séquence nucléotidique hétérologue comprend en outre une séquence peptidique d'adressage intracellulaire qui cible la pectate lyase sous forme soluble ou membranaire vers le réticulum endoplasmique ou les différents compartiments constitutifs du système endomembranaire de sécrétion de la cellule végétale.

7. Cellule végétale selon l'une des revendications 1 à 6, qui comprend un vecteur d'expression comprenant :
- des éléments d'ADN procaryote codant pour une origine de réplication bactérienne et un gène de résistance à un antibiotique ;
- la séquence nucléotidique hétérologue telle que définie dans l'une des revendications précédentes ;
- une cassette d'expression permettant l'expression d'un inhibiteur du silencing, de préférence p19 ; et
- des éléments d'ADN qui contrôlent le traitement des transcrits, comme des séquences de terminaison / polyadénylation, de préférence la séquence Tnos.

8. Plante comprenant au moins une cellule végétale selon l'une des revendications 1 à 7.

9. Procédé de production d'une pectate lyase choisie parmi Amb a 1, la sous-unité alpha d'Amb a 1, la sous-unité bêta d'Amb a 1 et les homologues d'Amb a 1, comprenant l'expression de ladite pectate lyase dans une cellule végétale selon l'une des revendications 1 à 7, ou dans une plante selon la revendication 8.

10. Procédé de production d'une pectate lyase selon la revendication 9, comprenant les étapes suivantes :
a) transformation d'agrobactéries avec un vecteur d'expression comprenant une séquence nucléotidique hétérologue codant pour une préproprotéine d'une pectate lyase liée de manière fonctionnelle à un promoteur fort ; et
b) transfection de la cellule végétale ou de la plante avec les agrobactéries obtenues à l'étape a).

11. Procédé de production d'une pectate lyase selon la revendication 10, dans lequel les agrobactéries utilisées dans l'étape a) sont choisies parmi les souches LBA4404, GV3101, EHA 101/105 et C58, et dans lequel la transfection de l'étape b) comprend de préférence les étapes suivantes :
b1) culture de la cellule végétale ou de la plante, en aéroponie ou en hydroponie, et sous éclairage LEDs, de préférence pendant cinq semaines en hydroponie sur des flotteurs libres,
b2) agroinfiltration de la cellule végétale ou plante obtenue en b1) sous vide, par les agrobactéries obtenues à l'étape a) ; et
b3) remise en culture de la cellule végétale ou de la plante obtenue en b2), typiquement pendant 3 à 6 jours.

12. Procédé de production d'une pectate lyase selon la revendication 10 ou 11, comprenant en outre une étape c) d'extraction de la pectate lyase produite, ladite étape c) comprenant les étapes suivantes :
- infiltration sous vide de la cellule végétale ou des feuilles (i.e. partie aérienne) de la plante, dans une solution enzymatique contenant une pectinase ou de la macérozyme, qui ne présente pas d'activité protéolytique,
- prélèvement de la cellule végétale infiltrée ou des feuilles infiltrées de la plante et incubation, dans une solution enzymatique de pectinase ou de macérozyme, pendant une durée comprise entre 2h30 et 5h, à une température comprise entre 24°C et 30°C, puis
- mise sous agitation du mélange obtenu entre 20 et 30 rpm à température ambiante pendant une durée comprise entre 30 minutes et 2h,
- filtration puis éventuellement centrifugation du digestat obtenu, et
récupération du surnageant.

## Patentansprüche

1. Pflanzliche Zelle, die ein DNA-Molekül umfasst, umfassend wenigstens eine heterologe Nukleotidsequenz, die für ein Präprotein einer Pektatlyase kodiert, ausgewählt aus Amb a 1 der Untereinheit alpha von Amb a 1, der Untereinheit beta von Amb a 1 und den Homologen von Amb a 1, funktionsfähig mit einem starken Promotor verbunden, wobei das genannte DNA-Molekül nicht in das Genom der pflanzlichen Zelle integriert ist.

2. Pflanzliche Zelle nach Anspruch 1, wobei die Pektatlyase unter Amb a 1.0101, Amb a 1.0201, Amb a 1.0202, Amb a 1.0301, Amb a 1.0302, Amb a 1,0303, Amb a 1.0304, Amb a 1,0305, Amb a 1.0401, Amb a 1.0402, Amb a 1.0501, Amb a 1.0502 der Untereinheit alpha von Amb a 1, der Untereinheit beta von Amb a 1 und den Proteinen der Familie von Asteracea ausgewählt ist, die wenigstens 57% Identität, vorzugsweise wenigstens 60% Identität, vorzugsweise wenigstens 64% Identität mit einer der Isoformen von Amb a 1 aufweisen.

3. Pflanzliche Zelle nach einem der Ansprüche 1 oder 2, wobei die Pektatlyase aus Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502 der Untereinheit alpha von Amb a 1, der Untereinheit beta von Amb a 1 und den Proteinen Artemisa vulgaris, Ambrosia psilostachya oder Ambrosia trifida ausgewählt ist, die wenigstens 57% Identität, vorzugsweise wenigstens 60% Identität, vorzugsweise wenigstens 64% Identität mit einem der Isoformen von Amb a 1 aufweisen.

4. Pflanzliche Zelle nach einem der Ansprüche 1 bis 3, wobei die heterologe Nukleotidsequenz eine Sequenz umfasst, die das Peptidsignal kodiert, eine Sequenz, die das Propeptid kodiert und eine Sequenz, die die reife Pektatlyase kodiert.

5. Pflanzliche Zelle nach Anspruch 4, wobei:
- das Peptidsignal aus dem natürlichen Peptidsignal der Pektatlyase und dem Peptidsignal der Tabakchitinase ausgewählt ist; und
- das Propeptid aus den natürlichen Propeptiden der Pektatlyasen und den Propeptiden der Peptidasen C1A ausgewählt ist.

6. Pflanzliche Zelle nach einem der Ansprüche 1 bis 5, wobei die heterologe Nukleotidsequenz darüber hinaus eine Peptidsequenz für die intrazelluläre Adressierung umfasst, die die Pektatlyase in eine lösliche oder membranförmige Form oder die verschiedenen konstituierenden Kammern des Endomembransystems der Sekretion der pflanzlichen Zelle umfasst.

7. Pflanzliche Zelle nach einem der Ansprüche 1 bis 6, die einen Expressionsvektor umfasst, umfassend:
- prokaryotische DNA-Elemente, die für einen bakteriellen Replikationsursprung kodieren und ein Antibiotikaresistenzgen kodieren;
- die heterologe Nukleotidsequenz, die nach einem der vorhergehenden Ansprüche definiert ist, eine Expressionskassette, die die Expression eines Silencing-Inhibitors, vorzugsweise p19, ermöglicht; und
- DNA-Elemente, die die Verarbeitung der Transkription steuern, wie Terminierung/Polyadenylierungssequenzen, vorzugsweise die Sequenz Tnos.

8. Pflanze, die wenigstens eine pflanzliche Zelle nach einem der Ansprüche 1 bis 7 umfasst.

9. Verfahren zur Herstellung einer Pektatlyase, die aus Amb a 1, der Untereinheit alpha von Amb a 1, der Untereinheit beta von Amb a 1 und den Homologen von Amb a 1 ausgewählt ist, umfassend die Expression der genannten Pektatlyase in eine pflanzliche Zelle nach einem der Ansprüche 1 bis 7 oder in einer Pflanze nach Anspruch 8.

10. Verfahren zur Herstellung einer Pektatlyase nach Anspruch 9, umfassend die folgenden Schritte:
a) Transformation von Agrobakterien mit einem Expressionsvektor, der eine heterologe Nukleotidsequenz umfasst, die für ein Präprotein einer Pektatlyase kodiert, funktionsfähig mit einem starken Promotor verbunden; und
b) Transfektion der pflanzlichen Zelle oder der Pflanze mit in Stufe a) erhaltenen Agrobakterien.

11. Herstellungsverfahren einer Pektatlyase nach Anspruch 10, wobei die verwendeten Agrobakterien in Stufe a) aus Stämmen LBA4404, GV3101, EHA 101/105 und C58 ausgewählt sind und wobei die Transfektion von Stufe b) vorzugsweise die folgenden Schritte umfasst:
b1) Kultur der pflanzlichen Zelle oder der Pflanze in Aeroponic oder Hydroponic und unter LED-Beleuchtung, vorzugsweise im Verlauf von fünf Hydroponic-Wochen auf freien Schwebekörpern;
b2) Agroinfiltration der pflanzlichen Zelle oder der erhaltenen Pflanze in b1) unter Vakuum, mit den in Schritt a) erhaltenen Agrobakterien; und
b3) Bereitstellen der pflanzen Kultur oder der in b2) erhaltenen Pflanze, typischerweise zwischen 3 bis 6 Stunden.

12. Verfahren zur Herstellung einer Pektatlyase nach Anspruch 10 oder 11, umfassend unter anderem einen Schritt c) der Extraktion des Pektatlyase-Produkts, wobei der genannte Schritt c) die folgenden Schritte umfasst:
- Infiltration im Vakuum der pflanzlichen Zelle oder der Blätter (d.h. oberirdisch) der Pflanze in einer Enzymlösung, die eine Pektinase oder von dem Macerozym enthält, das keine proteolytische Aktivität aufweist,
- Entnahme der infiltrierten pflanzlichen Zelle oder der infiltrierten Blätter der Pflanze und Inkubation in einer Enzymlösung von Pektidase oder von Macerozym während einem Zeitraum, der 2h30 und 5h umfasst, bei einer Temperatur, die zwischen 24°C und 30°C umfasst, danach
- Rühren der erhaltenen Mischung zwischen 20 und 30 U/min bei Raumtemperatur während einem Zeitraum, der 30 Minuten und 2h umfasst,
- Filtration und anschließende optionale Zentrifugation des erhaltenen Verdaus und Rückgewinnung des Überstands.

## Claims

1. A plant cell comprising a DNA molecule comprising at least one heterologous nucleotide sequence encoding a preproprotein of a pectate lyase chosen from Amb a 1, the alpha subunit of Amb a 1, the beta subunit of Amb a 1, and homologs of Amb a 1, functionally bound to a strong promoter, wherein said DNA molecule is not integrated into the plant cell genome.

2. The plant cell according to claim 1, wherein the pectate lyase is chosen from Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, the alpha subunit of Amb a 1, the beta subunit of Amb a 1 and proteins of the Asteraceae family which have at least 57% identity, preferably at least 60% identity, preferably at least 64% identity, with one of the isoforms of Amb a 1.

3. The plant cell according to one of claims 1 or 2, wherein the pectate lyase is chosen from Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, the alpha subunit of Amb a 1, the beta subunit of Amb a 1 and proteins of *Artemisia vulgaris, Ambrosia psilostachya* or *Ambrosia trifida* which have at least 57% identity, preferably at least 60% identity, preferably at least 64% identity, with one of the isoforms of Amb a 1.

4. The plant cell according to one of claims 1 to 3, wherein the heterologous nucleotide sequence comprises a sequence encoding the signal peptide, a sequence encoding the propeptide and a sequence encoding the mature pectate lyase.

5. The plant cell according to claim 4, wherein:
- the signal peptide is chosen from the natural pectate lyase signal peptide and the tobacco chitinase signal peptide; and
- the propeptide is chosen from the natural pectate lyase propeptides and the propeptides of peptidases C1A.

6. The plant cell according to one of claims 1 to 5, wherein the heterologous nucleotide sequence further comprises an intracellular trafficking peptide sequence which targets the pectate lyase in soluble or membrane form to the endoplasmic reticulum or the various compartments which constitute the endomembrane secretory system of the plant cell.

7. The plant cell according to one of claims 1 to 6, which comprises an expression vector comprising:
- elements of prokaryotic DNA encoding a bacterial origin of replication and a gene for antibiotic resistance;
- the heterologous nucleotide sequence as defined in one of the preceding claims;
- an expression cassette allowing the expression of a silencing suppressor, preferably p19; and
- DNA elements which control transcript processing, such as termination/polyadenylation sequences, preferably the Tnos sequence.

8. A plant comprising at least one plant cell according to one of claims 1 to 7.

9. A process for producing a pectate lyase chosen from Amb a 1, the alpha subunit of Amb a 1, the beta subunit of Amb a 1, and homologs of Amb a 1, comprising the expression of said pectate lyase in a plant cell according to one of claims 1 to 7, or in a plant according to claim 8.

10. The process for producing a pectate lyase according to claim 9, comprising the following steps:
a) transformation of agrobacteria with an expression vector comprising a heterologous nucleotide sequence encoding a preproprotein of a pectate lyase, functionally bound to a strong promoter; and
b) transfection of the plant cell or of the plant with the agrobacteria obtained in step a).

11. The process for producing a pectate lyase according to claim 10, wherein the agrobacteria used in step a) are chosen from the strains LBA4404, GV3101, EHA 101/105 and C58, and wherein the transfection of step b) preferably comprises the following steps:
b1) culturing the plant cell or plant aeroponically or hydroponically, under LED lighting, preferably for five weeks hydroponically on free floats,
b2) agroinfiltration of the plant cell or plant obtained in b1), under vacuum, by the agrobacteria obtained in step a); and
b3) returning the plant cell or plant obtained in b2) to culturing, typically for 3 to 6 days.

12. The process for producing a pectate lyase according to claim 10 or 11, further comprising a step c) of extraction of the pectate lyase produced, said step c) comprising the following steps:
- infiltration under vacuum of the plant cell or leaves (i.e. the aerial part) of the plant, in an enzymatic solution comprising a pectinase or maceroenzyme, which does not have proteolytic activity,
- taking off the infiltrated plant cell or infiltrated leaves from the plant and incubation, in an enzymatic solution of pectinase or maceroenzyme, for a duration of between 2 h 30 and 5 h, at a temperature of between 24°C and 30°C, then
- placing the mixture obtained under agitation at between 20 and 30 rpm at room temperature for a duration of between 30 minutes and 2 h,
- filtration then optional centrifugation of the digestate obtained, and
- recovery of the supernatant.


### SEQUENCE LISTING

<110> ANGANY GENETICS
<120> Production commerciale de l'allergène Amb A1 par expression transitoire chez les plantes
<130> BFF140239
<160> 9
<170> Patent In version 3.5
<210> 1
   <211> 1254
   <212> DNA
   <213> Artificial Sequence
<220>
   <223> ADNc codant pour la préproprotéine naturelle (de séquence SEQ ID NO :7)
<400> 1
   tctagaggta ccatggggat caaacactgt tgttacatct tgtattttac cttagccctt      60
   gtcactttgc tgcaacctgt tcgttctgcc gaagatctcc aggaaatctt accagttaac     120
   gaaacaagga ggctgacaac aagtggagca tacaacatta tagacgggtg ctggaggggc     180
   aaagccgatt gggcggaaaa ccgaaaagcg ttagccgatt gtgcccaagg ttttgggaag     240
   ggaacagtgg gcggaaaaga tggtgatata tacacggtca ccagtgagct agatgatgat     300
   gttgcaaatc caaaagaagg cacactccgg tttggtgccg cccaaaacag gcccttgtgg     360
   atcatttttg aaagagatat ggtgattcgt ttggataaag agatggtggt aaacagtgac     420
   aagaccatcg atggccgagg ggcgaaagtt gaaatcatta acgctggttt cacccttaat     480
   ggtgtcaaga atgtaatcat tcataacata aatatgcatg atgttaaagt gaatccagga     540
   ggcctgatta agtccaacga tggtccagca gctccaagag ctggtagtga tggtgatgct     600
   ataagtattt ctggtagttc acaaatatgg atcgaccatt gttcgctcag taagtctgtt     660
   gatgggctgg tagatgccaa gctcggcacc acacgcttaa ccgtttccaa cagcttattc     720
   acccaacacc agtttgtact attattcggg gctggtgacg aaaatattga agatagaggc     780
   atgctagcaa cggtcgcttt caacacgttc actgataacg ttgaccaaag aatgcctaga     840
   tgtcggcatg ggtttttcca agtcgttaac aacaactatg ataaatgggg atcgtatgcc     900
   atcggtggta gcgcgtcccc aaccatactc agccaaggga acagattctg cgcccccgat     960
   gaacgcagca agaaaaatgt cctaggaagg catggtgaag ccgccgcaga gtcgatgaag    1020
   tggaactgga gaacgaataa agacgtgctt gaaaatggtg ctatttttgt tgcatccggg    1080
   gtcgatccag tgctaacccc tgagcaaagc gcagggatga ttccagccga accaggagag    1140
   tccgctctaa gcctcactag tagtgctggt gtactctcat gccaacccgg agcaccttgc    1200
   catcatcatc atcatcatga ttataaagat gatgatgata aagtttgagt cgac            1254
<210> 2
   <211> 1254
   <212> DNA
   <213> Artificial Sequence
<220>
   <223> ADNc optimisé pour une utilisation chez N.benthamiana, codant pour la préproprotéine naturelle (de séquence SEQ ID NO :7)
<400> 2
   tctagaggta ccatgggcat caagcactgc tgctacatcc tctacttcac cctcgccctc      60
   gtcaccctcc tccagcccgt ccgctccgcc gaggacctcc aggagatcct ccccgtcaac     120
   gagacccgcc gcctcaccac ctccggcgcc tacaacatca tcgacggctg ctggcgcggc     180
   aaggccgact gggccgagaa ccgcaaggcc ctcgccgact gcgcccaggg cttcggcaag     240
   ggcaccgtcg gcggcaagga cggcgacatc tacaccgtca cctccgagct cgacgacgac     300
   gtcgccaacc ccaaggaggg caccctccgc ttcggcgccg cccagaaccg ccccctctgg     360
   atcatcttcg agcgcgacat ggtcatccgc ctcgacaagg agatggtcgt caactccgac     420
   aagaccatcg acggccgcgg cgccaaggtc gagatcatca acgccggctt caccctcaac     480
   ggcgtcaaga acgtcatcat ccacaacatc aacatgcacg acgtcaaggt caaccccggc     540
   ggcctcatca agtccaacga cggccccgcc gccccccgcg ccggctccga cggcgacgcc      600
   atctccatct ccggctcctc ccagatctgg atcgaccact gctccctctc caagtccgtc     660
   gatggcctcg tcgatgccaa gctcggcacc acccgcctca ccgtctccaa ctccctcttc     720
   acccagcacc agttcgtcct cctcttcggc gccggcgacg agaacatcga ggaccgcggc     780
   atgctcgcca ccgtcgcctt caacaccttc accgacaacg ttgaccagcg catgccccgc     840
   tgccgccacg gcttcttcca ggtcgtcaac aacaactacg acaagtgggg ctcctacgcc     900
   atcggcggct ccgcctcccc caccatcctc tcccagggca accgcttctg cgcccccgac     960
   gagcgctcca agaagaacgt cctcggccgc cacggcgagg ccgccgccga gtccatgaag    1020
   tggaactggc gcaccaacaa ggacgtcctc gagaacggcg ccatcttcgt cgcctccggc    1080
   gttgaccccg tcctcacccc cgagcagtcc gccggcatga tccccgccga gcccggcgag    1140
   tccgccctct ccctcacctc ctccgccggc gtcctctcct gccagcccgg cgccccctgc    1200
   catcatcatc atcatcatga ttataaagat gatgatgata aagtttgagt cgac           1254
<210> 3
   <211> 1254
   <212> DNA
   <213> Artificial Sequence
<220>
   <223> ADNc harmonisé codant pour la forme la préproprotéine naturelle (de séquence SEQ ID NO :7)
<400> 3
   tctagaggta ccatggggat taaacactgt tgttacattt tgtactttac tctcgcactc        60
   gtgaccctcc tccagcctgt gcgctccgca gaggatcttc aggagattct cccagttaat       120
   gagaccagga ggctgaccac ctccggagca tacaatatta tcgacggatg ctggagggga       180
   aaggcagatt gggccgagaa tcggaaggcc ctcgcagatt gcgcacaagg ttttggcaag       240
   ggaaccgtgg gaggaaagga tggtgatatc tacaccgtga cttccgagct cgatgatgat       300
   gttgcaaatc caaaggaggg aaccctccgc ttcggtgcag cacagaatag gcccctctgg       360
   attatttttg agagggatat ggtgattcgg ctcgataagg agatggtggt caattccgac       420
   aagactattg atggacgggg agccaaggtt gagattatta atgctggttt cactctcaat       480
   ggtgttaaga atgtcattat tcataatatc aatatgcacg atgttaaggt gaatcctgga       540
   ggactgatta agtccaatga tggtcctgca gctcctagag ctggttccga tggtgatgct       600
   atctccattt ccggttcctc ccagatctgg attgaccatt gttccctttc caagtccgtg       660
   gatggtctcg tcgatgctaa gcttggaact acccgcctca ctgtttccaa ttccctcttc       720
   actcagcacc agtttgtcct cctcttcggc gctggtgacg agaatattga ggataggggt       780
   atgctcgcaa ccgtggcttt caataccttc accgataatg ttgaccagag gatgcctagg       840
   tgccggcatg gatttttcca ggtggttaat aataattacg ataagtgggg atcctacgca       900
   attggtggtt ccgcctcccc tactatcctt tcccagggaa ataggttctg cgcacccgat       960
   gagcgctcca agaagaatgt gctcggaagg cacggtgagg cagcagcaga gtccatgaag      1020
   tggaattgga ggaccaataa ggacgtgctc gagaatggtg ctatttttgt tgcatccgga      1080
   gttgatcctg tgctcactcc tgagcagtcc gcaggaatga ttcctgcaga gcctggagag      1140
   tccgctctct cccttacctc ctccgctggt gtcctttcct gccagcccgg agcaccttgc      1200
   catcatcatc atcatcatga ttataaagat gatgatgata aagtttgagt cgac            1254
<210> 4
   <211> 606
   <212> DNA
   <213> Artificial Sequence
<220>
   <223> ADNc natif/optimisé/harmonisé codant pour la sous-unité bêta
<400> 4
   tctagaggta ccatggggat caaacactgt tgttacatct tgtattttac cttagccctt        60
   gtcactttgc tgcaacctgt tcgttctgcc gaagatctcc aggaaatctt accagttaac       120
   gaaacaagga ggctgacaac aagtggagca tacaacatta tagacgggtg ctggaggggc       180
   aaagccgatt gggcggaaaa ccgaaaagcg ttagccgatt gtgcccaagg ttttgggaag       240
   ggaacagtgg gcggaaaaga tggtgatata tacacggtca ccagtgagct agatgatgat 300
   gttgcaaatc caaaagaagg cacactccgg tttggtgccg cccaaaacag gcccttgtgg 360
   atcatttttg aaagagatat ggtgattcgt ttggataaag agatggtggt aaacagtgac 420
   aagaccatcg atggccgagg ggcgaaagtt gaaatcatta acgctggttt cacccttaat 480
   ggtgtcaaga atgtaatcat tcataacata aatatgcatg atgttaaagt gaatccagga 540
   ggcctgatta agcatcatca tcatcatcat gattataaag atgatgatga taaagtttga 600
   gtcgac 606
<210> 5
   <211> 786
   <212> DNA
   <213> Artificial Sequence
<220>
   <223> ADNc natif/optimisé/harmonisé codant pour la sous-unité alpha
<400> 5
   tctagaggta ccatggggat caaacactgt tgttacatct tgtattttac cttagccctt 60
   gtcactttgc tgcaacctgt tcgttctaac gatggtccag cagctccaag agctggtagt 120
   gatggtgatg ctataagtat ttctggtagt tcacaaatat ggatcgacca ttgttcgctc 180
   agtaagtctg ttgatgggct ggtagatgcc aagctcggca ccacacgctt aaccgtttcc 240
   aacagcttat tcacccaaca ccagtttgta ctattattcg gggctggtga cgaaaatatt 300
   gaagatagag gcatgctagc aacggtcgct ttcaacacgt tcactgataa cgttgaccaa 360
   agaatgccta gatgtcggca tgggtttttc caagtcgtta acaacaacta tgataaatgg 420
   ggatcgtatg ccatcggtgg tagcgcgtcc ccaaccatac tcagccaagg gaacagattc 480
   tgcgcccccg atgaacgcag caagaaaaat gtcctaggaa ggcatggtga agccgccgca 540
   gagtcgatga agtggaactg gagaacgaat aaagacgtgc ttgaaaatgg tgctattttt 600
   gttgcatccg gggtcgatcc agtgctaacc cctgagcaaa gcgcagggat gattccagcc 660
   gaaccaggag agtccgctct aagcctcact agtagtgctg gtgtactctc atgccaaccc 720
   ggagcacctt gccatcatca tcatcatcat gattataaag atgatgatga taaagtttga 780
   gtcgac 786
<210> 6
   <211> 80
   <212> PRT
   <213> Artificial Sequence
<220>
   <223> propeptide Der p1
<400> 6
   Arg Pro Ser Ser Ile Lys Thr Phe Glu Glu Tyr Lys Lys Ala Phe Asn
   1               5                   10                  15
   Lys Ser Tyr Ala Thr Phe Glu Asp Glu Glu Ala Ala Arg Lys Asn Phe
               20                  25                  30
   Leu Glu Ser Val Lys Tyr Val Gln Ser Asn Gly Gly Ala Ile Asn His
           35                  40                  45
   Leu Ser Asp Leu Ser Leu Asp Glu Phe Lys Asn Arg Phe Leu Met Ser
       50                  55                  60
   Ala Glu Ala Phe Glu His Leu Lys Thr Gln Phe Asp Leu Asn Ala Glu
   65                  70                  75                  80
<210> 7
   <211> 417
   <212> PRT
   <213> Ambrosia artemisiifolia
<400> 7
   Ser Arg Gly Thr Met Gly Ile Lys His Cys Cys Tyr Ile Leu Tyr Phe
   1               5                   10                  15
   Thr Leu Ala Leu Val Thr Leu Leu Gln Pro Val Arg Ser Ala Glu Asp
               20                  25                  30
   Leu Gln Glu Ile Leu Pro Val Asn Glu Thr Arg Arg Leu Thr Thr Ser
           35                  40                  45
   Gly Ala Tyr Asn Ile Ile Asp Gly Cys Trp Arg Gly Lys Ala Asp Trp
       50                  55                  60
   Ala Glu Asn Arg Lys Ala Leu Ala Asp Cys Ala Gln Gly Phe Gly Lys
   65                  70                  75                  80
   Gly Thr Val Gly Gly Lys Asp Gly Asp Ile Tyr Thr Val Thr Ser Glu
                   85                  90                  95
   Leu Asp Asp Asp Val Ala Asn Pro Lys Glu Gly Thr Leu Arg Phe Gly
               100                 105                 110
   Ala Ala Gln Asn Arg Pro Leu Trp Ile Ile Phe Glu Arg Asp Met Val
           115                 120                 125
   Ile Arg Leu Asp Lys Glu Met Val Val Asn Ser Asp Lys Thr Ile Asp
       130                 135                 140
   Gly Arg Gly Ala Lys Val Glu Ile Ile Asn Ala Gly Phe Thr Leu Asn
   145                 150                 155                 160
   Gly Val Lys Asn Val Ile Ile His Asn Ile Asn Met His Asp Val Lys
                   165                 170                 175
   Val Asn Pro Gly Gly Leu Ile Lys Ser Asn Asp Gly Pro Ala Ala Pro
               180                 185                 190
   Arg Ala Gly Ser Asp Gly Asp Ala Ile Ser Ile Ser Gly Ser Ser Gln
           195                 200                 205
   Ile Trp Ile Asp His Cys Ser Leu Ser Lys Ser Val Asp Gly Leu Val
       210                 215                 220
   Asp Ala Lys Leu Gly Thr Thr Arg Leu Thr Val Ser Asn Ser Leu Phe
   225                 230                 235                 240
   Thr Gln His Gln Phe Val Leu Leu Phe Gly Ala Gly Asp Glu Asn Ile
                   245                 250                 255
   Glu Asp Arg Gly Met Leu Ala Thr Val Ala Phe Asn Thr Phe Thr Asp
               260                 265                 270
   Asn Val Asp Gln Arg Met Pro Arg Cys Arg His Gly Phe Phe Gln Val
           275                 280                 285
   Val Asn Asn Asn Tyr Asp Lys Trp Gly Ser Tyr Ala Ile Gly Gly Ser
       290                 295                 300
   Ala Ser Pro Thr Ile Leu Ser Gln Gly Asn Arg Phe Cys Ala Pro Asp
   305                 310                 315                 320
   Glu Arg Ser Lys Lys Asn Val Leu Gly Arg His Gly Glu Ala Ala Ala
                   325                 330                 335
   Glu Ser Met Lys Trp Asn Trp Arg Thr Asn Lys Asp Val Leu Glu Asn
               340                 345                 350
   Gly Ala Ile Phe Val Ala Ser Gly Val Asp Pro Val Leu Thr Pro Glu
           355                 360                 365
   Gln Ser Ala Gly Met Ile Pro Ala Glu Pro Gly Glu Ser Ala Leu Ser
       370                 375                 380
   Leu Thr Ser Ser Ala Gly Val Leu Ser Cys Gln Pro Gly Ala Pro Cys
   385                 390                 395                 400
   His His His His His His Asp Tyr Lys Asp Asp Asp Asp Lys Val Val
   405 410 415
   Asp
<210> 8
   <211> 201
   <212> **PRT**
   <213> Ambrosia artemisiifolia
<400> 8
   Ser Arg Gly Thr Met Gly Ile Lys His Cys Cys Tyr Ile Leu Tyr Phe
   1 5 10 15
   Thr Leu Ala Leu Val Thr Leu Leu Gln Pro Val Arg Ser Ala Glu Asp
   20 25 30
   Leu Gln Glu Ile Leu Pro Val Asn Glu Thr Arg Arg Leu Thr Thr Ser
   35 40 45
   Gly Ala Tyr Asn Ile Ile Asp Gly Cys Trp Arg Gly Lys Ala Asp Trp
   50 55 60
   Ala Glu Asn Arg Lys Ala Leu Ala Asp Cys Ala Gln Gly Phe Gly Lys
   65 70 75 80
   Gly Thr Val Gly Gly Lys Asp Gly Asp Ile Tyr Thr Val Thr Ser Glu
   85 90 95
   Leu Asp Asp Asp Val Ala Asn Pro Lys Glu Gly Thr Leu Arg Phe Gly
   100 105 110
   Ala Ala Gln Asn Arg Pro Leu Trp Ile Ile Phe Glu Arg Asp Met Val
   115 120 125
   Ile Arg Leu Asp Lys Glu Met Val Val Asn Ser Asp Lys Thr Ile Asp
   130 135 140
   Gly Arg Gly Ala Lys Val Glu Ile Ile Asn Ala Gly Phe Thr Leu Asn
   145 150 155 160
   Gly Val Lys Asn Val Ile Ile His Asn Ile Asn Met His Asp Val Lys
   165 170 175
   Val Asn Pro Gly Gly Leu Ile Lys His His His His His His Asp Tyr
   180 185 190
   Lys Asp Asp Asp Asp Lys Val Val Asp
           195                 200
<210> 9
   <211> 261
   <212> PRT
   <213> Ambrosia artemisiifolia
<400> 9
   Ser Arg Gly Thr Met Gly Ile Lys His Cys Cys Tyr Ile Leu Tyr Phe
   1               5                   10                  15
   Thr Leu Ala Leu Val Thr Leu Leu Gln Pro Val Arg Ser Asn Asp Gly
               20                  25                  30
   Pro Ala Ala Pro Arg Ala Gly Ser Asp Gly Asp Ala Ile Ser Ile Ser
           35                  40                  45
   Gly Ser Ser Gln Ile Trp Ile Asp His Cys Ser Leu Ser Lys Ser Val
       50                  55                  60
   Asp Gly Leu Val Asp Ala Lys Leu Gly Thr Thr Arg Leu Thr Val Ser
   65                  70                  75                  80
   Asn Ser Leu Phe Thr Gln His Gln Phe Val Leu Leu Phe Gly Ala Gly
                   85                  90                  95
   Asp Glu Asn Ile Glu Asp Arg Gly Met Leu Ala Thr Val Ala Phe Asn
               100                 105                 110
   Thr Phe Thr Asp Asn Val Asp Gln Arg Met Pro Arg Cys Arg His Gly
           115                 120                 125
   Phe Phe Gln Val Val Asn Asn Asn Tyr Asp Lys Trp Gly Ser Tyr Ala
       130                 135                 140
   Ile Gly Gly Ser Ala Ser Pro Thr Ile Leu Ser Gln Gly Asn Arg Phe
   145                 150                 155                 160
   Cys Ala Pro Asp Glu Arg Ser Lys Lys Asn Val Leu Gly Arg His Gly
                   165                 170                 175
   Glu Ala Ala Ala Glu Ser Met Lys Trp Asn Trp Arg Thr Asn Lys Asp
               180                 185                 190
   Val Leu Glu Asn Gly Ala Ile Phe Val Ala Ser Gly Val Asp Pro Val
           195                 200                 205
   Leu Thr Pro Glu Gln Ser Ala Gly Met Ile Pro Ala Glu Pro Gly Glu
       210                 215                 220
   Ser Ala Leu Ser Leu Thr Ser Ser Ala Gly Val Leu Ser Cys Gln Pro
   225                 230                 235                 240
   Gly Ala Pro Cys His His His His His His Asp Tyr Lys Asp Asp Asp
                   245                 250                 255
   Asp Lys Val Val Asp
               260